# EUROPEAN PATENT SPECIFICATION

(11) **EP 0 413 538 B1**
(45) Date of publication and mention of the grant of the patent: **13.10.1993**
(21) Application number: 90308881.3
(22) Date of filing: 13.08.1990
(51) Int. Cl.: A61K 31/71, A61K 9/00, A61K 47/14

(54) **Long active injectable formulations containing triacetin**
Triacetin enthaltende, langwirkende injizierbare Zusammensetzungen
Compositions injectables à effet prolongé à base de triacétine

(30) Priority: 14.08.1989 US 393356
(43) Date of publication of application: 20.02.1991
(73) Proprietor: Merck & Co., Inc., Rahway New Jersey 07065-0900 (US)
(72) Inventor: Williams, James B., Freehold, NJ 07728 (US)
(74) Representative: Cole, William Gwyn

(56) References cited:
- FR-A- 2 570 390
- GB-A- 2 176 182

## Description

The avermectin series of compounds are potent anthelmintic and antiparasitic agents against internal and external parasites. The natural product avermectins are disclosed in US-A-4,310,519 to Albers Schonberg et al., and the 22,23-dihydro avermectin compounds are disclosed in Chabala et al, US-A- 4,199,569.

Triacetin (Triacetyl glycerine) is a known formulating agent which is generally recognized as safe by the US Food and Drug Administration. See Fragrance Raw Material Monographs pg 879-881 and Pattys Industrial Hygiene and Toxicology 3rd Ed. Vol 2A "Toxicology" Clayton et al, (Ed) pg 2321-2322. Triacetin has been disclosed as a stabilizing agent for prostaglandin compounds. See Yalkowsky US-A- 4,301,175 and 3,966,962 and Yalkowsky et al, Journal of Pharmaceutical Sciences, 68, pg 114-115 (1979). Triacetin has also been disclosed as a component of a long acting antiperspirant composition for topical use, see Claukin US-A- 4,548,808; and in a formulation for the transdermal administration of psychostimulants, see Mahjour et al, US-A-4,666,926.

This invention is concerned with the unexpectedly long duration of activity which is observed when avermectin injectable formulations are prepared using triacetin. Thus, it is an object of this invention to describe such a prolonged therapeutic effect. An additional object is to describe the avermectin compounds which may be employed in the long acting formulation. Additional objects will become apparent for a reading of the following description.

This invention consists of 24 injectable formulation of triacetin and an avermectin compound which has been found to have a considerably prolonged duration of activity against internal and external parasites.

Triacetin is the triacetic acid ester of glycerin, also referred to as glyceryl triacetate which has the following formula:

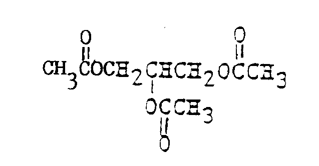

The avermectin compounds may have the following structure: 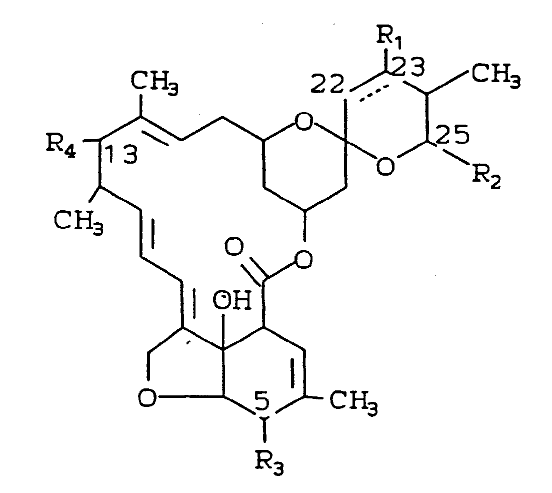
wherein the broken line indicates a single or a double bond at the 22,23-positions;
R₁ is hydrogen or hydroxy provided that R₁ is present only when the broken line indicates a single bond;
R₂ is alkyl of from 1 to 6 carbon atoms or alkenyl of from 3 to 6 carbon atoms;
R₃ is hydroxy, methoxy or = NOR₅ wherein R₅ is hydrogen or lower alkyl; and
R₄ is hydrogen, hydroxy or 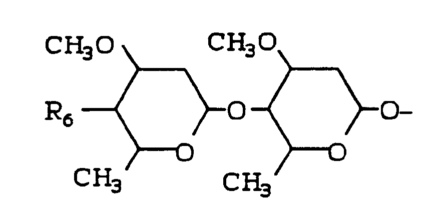
wherein R₆ is hydroxy, amino, mono-or di-loweralkyl amino or loweralkanoylamino.

The preferred compounds are avermectin B1a/B1b (abamectin) and 22,23-dihydro avermectin B1a/B1b (ivermectin). Both abamectin and ivermectin are approved as broad spectrum antiparasitic agents. The structures of abamectin and ivermectin are as follows: 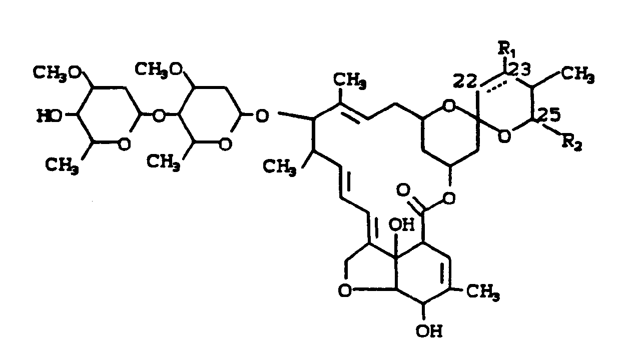
wherein for abamectin the broken line represents a double bond and R₁ is not present and for ivermectin the double bond represents a single bond and R₁ is hydrogen; and
R₂ is isopropyl or sec-butyl.

The abamectin and ivermectin products are generally prepared as a mixture of at least 80% of the compound wherein R₂ is sec-butyl and no more than 20% of the compound where R₂ is isopropyl.

In the injectable forms of the avermectin formulation it has not been possible to provide a long acting formulation since the avermectins are insoluble in water and generally of low solubility in the oil carriers usually used for injection. Heretofore, injectable formulations of a maximum of 14 days have been possible. The current long acting formulations of avermectins provided for unacceptably large volumes of the injection liquid, or resulted in irritation at the injection site.

The instant formulations of an avermectin compound with triacetin provides the advantages of a readily injectable formulation which provides the animal with treatment and protection against internal and external parasites for up to 42 days. The injection material is maintained as a depot to provide the necessary reservoir of active ingredient for a long acting effect. No tissue damage has been observed at the site of injection either at the time of injection or during the 42 day course of therapy. In contrast to most oil based formulations, the formulation maintains a low viscosity even at high drug loads and even at temperatures as low as -5°C, a temperature at which most oil-based formulations become too viscous for practical use. The high solubility of the avermectins in triacetin, allows for a low dose volume, as low as 1 ml per 50 kg of body weight. Previous oil formulations provide a lowest dose volume of 1 ml per 30 kg of body weight. Since previous injectable formulations only provide 14 days duration, the improved current formulation with 42 days duration avoids two injections which would be necessary to obtain 42 days protection with a 14 day duration injection and also avoids the stress of the extra handling necessary for the extra injections which increses illness and weight loss.

The triacetin formulation can contain the avermectin compound and the triacetin as the only ingredients. The formulations will generally be prepared to administer from 0.1 to 1 mg/kg preferably from 0.4 to 0.85 mg/kg and most preferably from about 0.6 to 0.7 mg/kg. At a preferred dose volume of about 1 ml to treat 50 kg of animal body weight the formulation contains from 5 to 50 mg of avermectin compound per ml of solution or about 0.5 to 10% w/v preferably 2.5 to 5% w/v.

In addition to the triacetin and the avermectin the formulation can contain an antioxidant such as a propyl gallate, BHA (butylated hydroxy anisole), BHT, (butylated hydroxy toluene) and monothioglycerol. The antioxidants are generally added to the formulation at rates of from 0.01 to 2.0% (w/v).

The formulation is prepared by dissolving the avermectin compound in approximately 80% of the intended volume of the triacetin. The antioxidant is then dissolved in the triacetin/avermectin mixture and the volume adjusted to 100% by the addition of the final volume of triacetin.

Since the long acting formulation is intended for injection, it is necessary that it be sterilized. Heat sterilization is generally to be avoided since the avermectin compound would be unstable at autoclave temperatures. Rather, membrane sterilization is preferred. The sterile solution is then packaged a septically.

The avermectin-triacetin long acting injectable formulation may be administered to warm blooded animals to provide up to 42 days of treatment and protection against external and internal parasites. Typically, the formulation is administered to domesticated animals such as cattle, sheep, pigs and horses.

### EXAMPLE OF THE INVENTION

An ivermectin (22,23-dihydro avermectin B1a/B1b in a ratio of 80:20) formulation in triacetin for injection, 3.15% is prepared from the following ingredients.
- Ivermectin: 3.15% w/v
- n-Propyl gallate: 0.02% w/v
- Triacetin: q.s. 100% w/v

The ivermectin is dissolved in approximately 80% of the triacetin. When dissolved, the n-propyl gallate is added and dissolved. The volume is adjusted to 100% with triacetin and the solution sterilized by membrane filtrations and packaged aseptically.

## Claims

1. A long acting injectable formulation consisting of an avermectin compound as the only active agent and of triacetin as the only solvent.

2. The formulation according to Claim 1 wherein the avermectin compound has the formula: 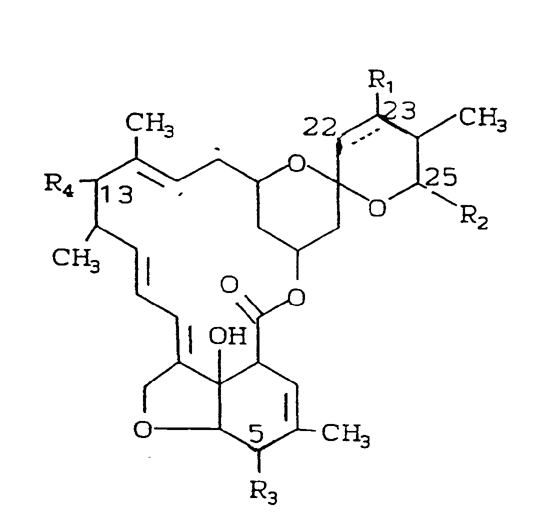 wherein the broken line indicates a single or a double bond at the 22,23-positions;
R₁ is hydrogen or hydroxy provided that R₁ is present only when the broken line indicates a single bond;
R₂ is alkyl of from 1 to 6 carbon atoms or alkenyl of from 3 to 6 carbon atoms;
R₃ is hydroxy, methoxy or = NOR₅ wherein R₅ is hydrogen or loweralkyl; and
R₄ is hydrogen, hydroxy or 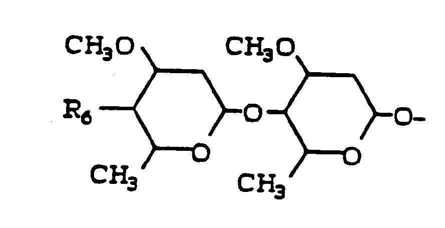 wherein R₆ is hydroxy, amino, mono- or di-loweralkyl amino or loweralkanoylamino.

3. The formulation according to Claim 2 wherein the avermectin compound has the formula: 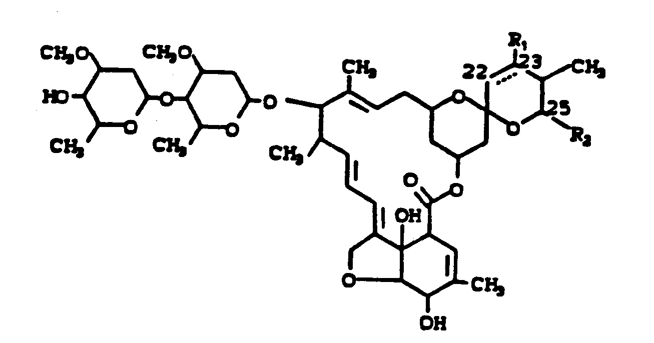 wherein for abamectin the broken line represents a double bond and R₁ is not present and for ivermectin the double bond represents a single bond and R₁ is hydrogen; and
R₂ is isopropyl or sec-butyl.

4. The formulation according to Claim 3 wherein the avermectin compound is ivermectin.

5. The formulation according to Claim 3 wherein the avermectin compound is abamectin.

6. The formulation according to Claim 1 which contains from 5 to 50 mg of the avermectin compound per ml of formulation.

7. The formulation according to Claim 1 which contains from 0.5 to 10% w/v of the avermectin compound.

8. A process for the preparation of the formulation according to Claim 1 which comprises dissolving the avermectin compound in about 80% of the volume of the triacetin; adding the remainder of the triacetin; and sterilizing the resultant formulation.

## Patentansprüche

1. Langwirkende injizierbare Formulierung, bestehend aus einer Avermectin-Verbindung als einzigem aktiven Mittel und Triacetin als einzigem Lösungsmittel.

2. Formulierung nach Anspruch 1, worin die Avermectin-Verbindung folgende Formel aufweist: 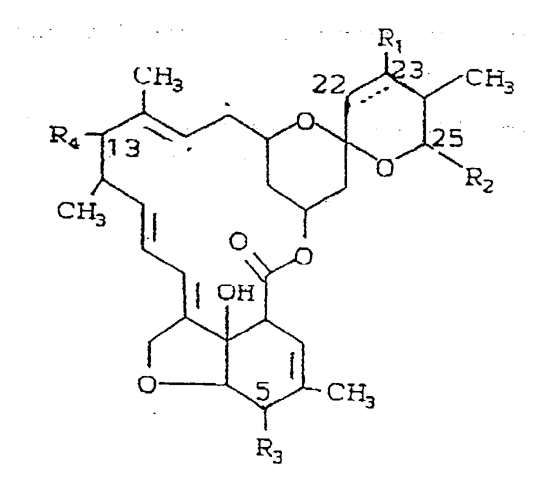 worin die durchbrochene Linie eine Einfach- oder Doppelbindung zwischen den Positionen 22, 23 bedeutet;
R₁ Wasserstoff oder Hydroxy darstellt, mit der Maßgabe, daß R₁ nur vorhanden ist, wenn die durchbrochene Linie eine Einfachbindung darstellt;
R₂ Alkyl mit 1 bis 6 Kohlenstoffatomen oder Alkenyl mit 3 bis 6 Kohlenstoffatomen bedeutet;
R₃ für Hydroxy, Methoxy oder =NOR₅ steht, worin R₅ Wasserstoff oder Niedrigalkyl darstellt; und
R₄ Wasserstoff oder Hydroxy oder 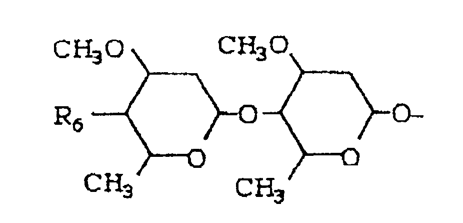 bedeutet; worin R₆ Wasserstoff, Amino, Mono- oder Di-Niedrigalkyl, Amino oder Niedrigalkanoylamino bedeutet;

3. Formulierung nach Anspruch 2, worin die Avermectin-Verbindung folgende Formel aufweist: 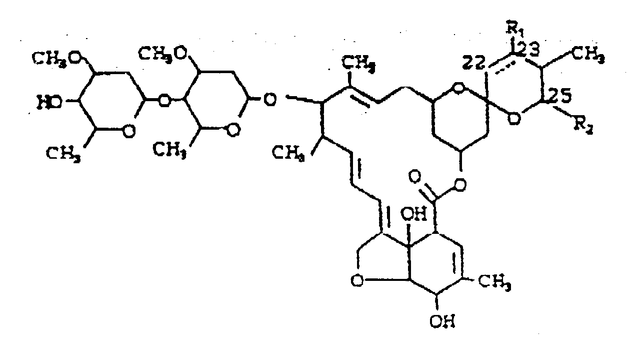 worin die durchbrochene Linie für Abamectin eine Doppelbindung darstellt und R₁ nicht vorhanden ist und die Doppelbindung bei Ivermectin eine Einfachbindung darstellt und R₁ für Wasserstoff steht; und
R₂ Isoprapyl oder sec-Butyl bedeutet.

4. Formulierung nach Anspruch 3, worin die Avermectin-Verbindung Ivermectin darstellt.

5. Formulierung nach Anspruch 3, worin die Avermectin-Verbindung Abamectin darstellt.

6. Formulierung nach Anspruch 1, die 5 bis 50 mg der Avermectin-Verbindung pro ml Formulierung enthält.

7. Formulierung nach Anspruch 1, die 0,5 bis 10 % (Gew./Vol.) der Avermectin-Verbindung enthält.

8. Verfahren zur Herstellung der Formulierung nach Anspruch 1, welches umfaßt Auflösen der Avermectin-Verbindung in etwa 80 % des Volumens von Triacetin; Hinzufügen des Restes von Triacetin; und Sterilisieren der resultierenden Formulierung.

## Revendications

1. Composition injectable à action prolongée, consistant en un composé de type avermectine en tant que seul principe actif et en triacétine en tant que seul solvant.

2. Composition selon la revendication 1, dans laquelle le composé de type avermectine est de formule: 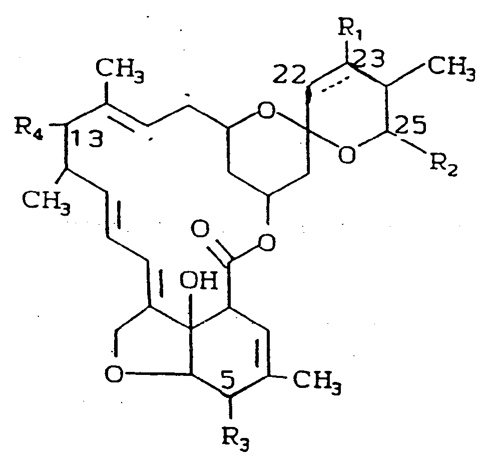 dans laquelle le trait interrompu représente une simple ou double liaison aux positions 22,23;
R₁ est un atome d'hydrogène ou le groupe hydroxy, étant entendu que R₁ n'est présent que lorsque le trait interrompu représente une simple liaison;
R₂ est un groupe alkyle ayant de 1 à 6 atomes de carbone ou alcényle ayant de 3 à 6 atomes de carbone;
R₃ est un groupe hydroxy, méthoxy ou =NOR₅, R₅ étant un atome d'hydrogène ou un radical alkyle inférieur; et
R₄ est un atome d'hydrogène ou un groupe hydroxy ou 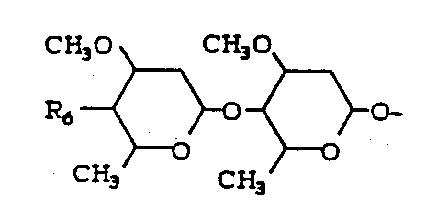 dans lequel R₆ est un groupe hydroxy, amino, mono- ou di-(alkyl inférieur)-amino ou (alcanoyl inférieur)-amino.

3. Composition selon la revendication 2, dans laquelle le composé de type avermectine est de formule: 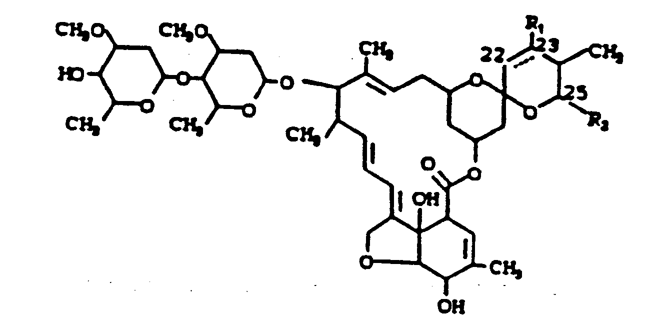 dans laquelle, pour l'abamectine, le trait interrompu représente une double liaison et R₁ n'est pas présent, et, pour l'ivermectine, le trait interrompu représente une simple liaison et R₁ est un atome d'hydrogène; et
R₂ est le groupe isopropyle ou sec-butyle.

4. Composition selon la revendication 3, dans laquelle le composé de type avermectine est l'ivermectine.

5. Composition selon la revendication 3, dans laquelle le composé de type avermectine est l'abamectine.

6. Composition selon la revendication 1, contenant de 5 à 50 mg du composé de type avermectine par ml de la composition.

7. Composition selon la revendication 1, contenant de 0,5 à 10 % p/v du composé de type avermectine.

8. Procédé pour la préparation de la composition selon la revendication 1, comprenant la dissolution du composé de type avermectine dans environ 80 % du volume de la triacétine; l'addition du reste de la triacétine; et la stérilisation de la composition résultante.
